# EUROPÄISCHE PATENTSCHRIFT

(11) **EP 1 246 945 B1**
(45) Veröffentlichungstag und Bekanntmachung des Hinweises auf die Patenterteilung: **23.06.2010**
(21) Anmeldenummer: 01907339.4
(22) Anmeldetag: 09.01.2001
(51) Int. Cl.: C12Q 1/68, G01N 33/48

(54) **VERFAHREN ZUR IDENTIFIZIERUNG EINER AUF EINEN KÖRPER AUFGEBRACHTEN MARKIERUNG**
METHOD FOR IDENTIFYING A MARK APPLIED ON A SOLID BODY
PROCEDE D'IDENTIFICATION D'UN MARQUAGE APPLIQUE SUR UN CORPS SOLIDE

(30) Priorität: 10.01.2000 DE 10000629
(43) Veröffentlichungstag der Anmeldung: 09.10.2002
(73) Patentinhaber: Secutech International Pte. Ltd., Singapore 069443 (SG)
(72) Erfinder: BERTLING, Wolf, 91056 Erlangen (DE); KOSAK, Hans, 53123 Bonn (DE); BAUER, Georg, 90409 Nürnberg (DE); WALTER, Harald, 8810 Horgen (CH); JOSTEN, André, 91334 Hemhofen (DE)
(74) Vertreter: UEXKÜLL & STOLBERG
(86) Internationale Anmeldenummer: PCT/DE2001/000055
(87) Internationale Veröffentlichungsnummer: WO 2001/051652

(56) Entgegenhaltungen:
- EP-A2- 0 745 690
- WO-A-87/06383
- DE-A- 19 811 730
- US-A- 5 139 812

### SEQUENZPROTOKOLL

<110> november AG
<120> Verfahren zur Identifizierung einer auf einen festen Körper aufgebrachten Markierung
<130> 401552
<140>
   <141>
<160> 3
<170> PatentIn Ver. 2.1
<210> 1
   <211> 33
   <212> DNA
   <213> Künstliche Sequenz
<220>
   <223> 5'-Amino
<220>
   <223> 5'-Amino markiertes Oligonukleotid
<400> 1
   tccaagcctg gagggatgat actttgcgct tgg 33
<210> 2
   <211> 33
   <212> DNA
   <213> Künstliche Sequenz
<220> '
   <223> 5'-Amino
<220>
   <223> 5'-Amino markiertes Oligonukleotid
<400> 2
   ttggaatcca tggttaaact tgtactttag gtc 33
<210> 3
   <211> 32
   <212> DNA
   <213> Künstliche Sequenz
<220>
   <223> 5'-6FAM; 3'dt(C2-DABCYL)
<220>
   <223> Beschreibung der künstlichen Sequenz: 5'-6FAM und 3'dt(C2-DABCYL) markiertes Oligonukleotid
<400> 3
   ccaagcgcaa agtatcatcc ctccaggctt gg 32

## Beschreibung

Die Erfindung betrifft ein Verfahren zur Identifizierung einer auf einen festen Körper aufgebrachten aus Flächenelementen gebildeten Markierung, einen Träger und einen Kit.

Die Erfindung betrifft insbesondere das Gebiet der Sicherheits-, Kodierungs- und Identifizierungstechnik.

Aus der DE 197 38 816 A1 ist es bekannt, an einem Feststoff zur Markierung gebundene Nukleinsäuren von dem Feststoff zu extrahieren oder zu entfernen. Die Nukleinsäuren werden in Lösung gebracht. Sie werden mittels einer spezifischen Reaktion, wie der PCR, vervielfältigt. Anschließend wird die vervielfältigte Nukleinsäuresequenz analysiert. Das Verfahren ist zeitaufwendig. Eine Extraktion der zur Markierung aufgebrachten Nukleinsäure ist nicht bei jedem Feststoff möglich oder erwünscht.

Ein Verfahren zum Identifizieren einer an einem Festkörper vorgesehenen Markierung ist aus der DE 198 11 730 A1 bekannt. Die Markierung weist hier eine Nukleotidsequenz auf. Die Nukleotidsequenz wird mit einer korrespondierenden Nukleotidsequenz in Kontakt gebracht, die an eine feste Phase eines Detektionsmittels gebunden ist. Für eine zufriedenstellende Hybridisierung muß die feste Phase des Detektionsmittels gegen die Markierung gedrückt werden. Das erschwert die Identifikation.

Aus der US 5,139,812 ist es bekannt, eine vorgegebene Nukleinsäure enthaltende Tinte zur fälschungssicheren Markierung von Gegenständen zu verwenden. Um eine Mehrzahl von Gegenständen unterscheidbar zu markieren, werden mit der Tinte unterschiedliche Beschriftungen aufgebracht. Die Identifikation einer solchermaßen aufgebrachten Markierung erfolgt durch Bindung einer weiteren Nukleinsäure an die vorgegebene Nukleinsäure. Die gebundene Nukleinsäure kann mittels einer Farbreaktion oder aufgrund einer radioaktiven Markierung sichtbar gemacht werden. Die Markierung kann durch eine sequenzunspezifische Nukleinsäurebindung ohne Kenntnis der zur Markierung verwendeten Sequenz dargestellt werden. Das Verfahren ist unsicher.

Die EP 0 745 690 A2 beschreibt sogenannte "molecular beacons" und deren Anwendung für die Hybridisierung. Eine Verwendung zur Detektion von Markierungen ist aus diesem Dokument nicht bekannt.

Die US 5,866,336 beschreibt mit einem Fluorophor markierte Primer. Die Primer werden mittels der Polymerase-Kettenreaktion hybridisiert. Im hybridisierten Zustand wird eine Rückfaltung der Primer aufgelöst. Das Fluoreszenzverhalten des am Primer vorgesehenen Fluorophors ändert sich damit. Das bekannte Verfahren ist für eine schnelle Identifikation einer Markierung ungeeignet, weil es die kosten- und zeitaufwendige Polymerase-Kettenreaktion erfordert.

Die DE 199 01 761 offenbart ein Verfahren zum Nachweis der Hybridisierung von DNA mittels Änderung eines RedoxPotentials. Eine solche Änderung des Redox-Protentials ist nicht ohne weiteres zu erfassen. Das bekannte Verfahren erlaubt keine schnelle und einfache Identifikation einer Markierung.

WO 87/06383 betrifft ein Verfahren zur Identifizierung eines Products mittels Hybridisierung auf einer Membran.

Aufgabe der vorliegenden Erfindung ist es, die Nachteile nach dem Stand der Technik zu beseitigen. Insbesondere soll ein alternatives Verfahren angegeben werden, mit dem eine sichere Identifizierung einer auf einem festen Körper aufgebrachten Markierung, schnell und einfach möglich ist.

Die Aufgabe wird durch die Merkmale des Anspruchs 1 gelöst. Zweckmäßige Ausgestaltungen der Erfindung ergeben sich aus den Merkmalen der Ansprüche 2 bis 18.

Die Erfindung befrifft somit ein Verfahren zur Identifizierung eines markierten Produkts, wobei das Produkt ein fester Körper ist und wobei eine auf den festen Körper (30) aufgebrachte aus Flächenelementen (10) gebildete vorgegebene Markierung (32), bei der an einen ersten Teil der Flächenelemente (10) erste Nukleinsäuren gebunden sind, so daß ein erstes vorgegebenes Teilmuster gebildet ist, mit folgenden Schritten identifiziert wird:
a) Inkontaktbringen der Markierung (32) mit in einer Lösung enthaltenen, zu den ersten Nukleinsäuren affinen dritten Nukleinsäuren, so daß die ersten und die dritten Nukleinsäuren hybridisieren, wobei die dritten Nukleinsäuren durch Auftropfen, Aufsaugen, Sprühen oder Zerstäuben homogen verteilt mit der Markierung (32) in Kontakt gebracht werden, und
b) Identifizierung des durch die hybridisierten ersten und dritten Nukleinsäuren gebildeten ersten Teilmusters auf dem festen Körper durch Nachweis der Hybridisierung zwischen den ersten und den dritten Nukleinsäuren mittels eines einstufigen Detektionsverfahrens, indem eine Änderung einer Fluoreszerizeigenschaft eines an der ersten oder dritten Nukleinsäure gebunden Fluorophors ohne Waschschritte detektiert wird
   wobei die Markierung direkt auf dem markierten Produkt identifiziert wird, ohne daß sie davon gelöst wird.

Die Nukleinsäuren können kovalent oder nicht-kovalent an den Flächenelementen gebunden sein. Sie können auch direkt an den Flächenelementen synthetisiert werden. Nukleinsäuren sind zu anderen Nukleinsäuren affin, wenn sie an diese spezifisch binden können.

Das erfindungsgemäße Verfahren ermöglicht eine sichere Identifizierung einer auf einen festen Körper aufgebrachten Markierung. Es ist insbesondere möglich, die Markierung direkt auf dem Produkt zu identifizieren, ohne daß sie davon gelöst werden muß.

Nach einer vorteilhaften Ausgestaltung wird vor dem Schritt lit. b der folgende Schritt durchgeführt: Binden zweiter Nukleinsäuren an einen zweiten Teil der Flächenelemente, so daß ein zweites Teilmuster gebildet ist Die Flächenelemente mit daran gebundenen zweiten Nukleinsäuren verhindern eine unspezifische Identifizierung, von Flächenelementen mit daran gebundenen ersten Nukleinsäuren Unter entsprechenden Bedingungen können unspezifische weitere dritte Nukleinsäuren an Flächenelementen mit daran gebundenen ersten Nukleinsäuren binden. Dabei binden sie jedoch auch an alle anderen Flächenelemente und ermöglichen keine Identifizierung der Markierung. Eine solche Identifizierung ist nur möglich, wenn die zugehörigen spezifischen dritten Nukleinsäuren bekannt sind. Dadurch ist das Verfahren sehr sicher. Das Verfahren läßt sich außerdem schnell und einfach durchführen.

Die Nukleinsäuren können, insbesondere synthetische und/oder einzelsträngige, Nukleinsäuren oder deren Analoga sein.

Bei einer vorteilhaften Ausgestaltung werden beim Schritt lit. b) zusätzlich vierte, zu den zweiten Biopolymeren affine Nukleinsäuren mit der Markierung in Kontakt gebracht. Beim Schritt lit. c) werden die Bindungen zwischen den zweiten und den vierten Nukleinsäuren nachgewiesen und die durch die gebundenen zweiten und vierten Nukleinsäuren gebildeten Teilmuster werden identifiziert. Der Nachweis der zweiten Nukleinsäuren ist nur möglich, wenn die zugehörigen spezifischen vierten Nukleinsäuren bekannt sind. Ein solches Verfahren ist sicherer als ein Verfahren, bei dem nur eine erste Nukleinsäure spezifisch identifiziert wird. Zwischen dem durch die gebundenen ersten und dritten Nukleinsäuren gebildeten Teilmuster und dem durch die gebundenen zweiten und vierten Nukleinsäuren gebildeten Teilmuster kann ein deutlicher Kontrast erzeugt werden. Dazu können die dritten und vierten Nukleinsäuren mit deutlich unterscheidbaren Markierungssubstanzen versehen sein. Die Trennschärfe zwischen den Teilmustern ist deutlich höher als beim bloßen Nachweis des durch die gebundenen ersten und dritten Nukleinsäuren gebildeten Teilmusters. Dies ist besonders vorteilhaft, wenn das Teilmuster sehr kleim oder schmal ist. Die dritten und ggf. die vierten Nukleinsäuren können in einer Lösung enthalten sein. Dadurch wird eine einfache Handhabung des Verfahrens gewährleistet.

Bei einer weiteren vorteilhaften Ausgestaltung wird das Inkontaktbringen unter vorgegeben stringenten Bindungsbedingungen, vorzugsweise bei Raumtemperatur, durchgeführt. Stringente Bindungsbedingungen sind Bedingungen, unter denen die dritten und ggf. die vierten Nukleinsäuren im wesentlichen nur an diejenigen Nukleinsäuren binden, zu denen sie affin sind. Unspezifische Bindung an andere Nukleinsäuren finden im wesentlichen nicht statt. Stringente Bindungsbedingungen können durch eine entsprechende Temperatur oder Ionenstärke erreicht werden. Bei Nukleinsäuren können die stringenten Bindungsbedingungen durch die Wahl entsprechender Nukleotidsequenzen bestimmt werden. So ist eine Anpassung der stringenten Bindungsbedingungen an den jeweiligen Zweck der Markierung möglich. Vorteilhaft ist es, wenn sich die Nukleinsäuren in ihren Nukleotidsequenzen möglichst weitgehend unterscheiden. So sind unspezifische Hybridisierungen sehr unwahrscheinlich.

Vorteilhafterweise wird mindestens ein weiteres Biopolymer oder das zweite Nukleinsäure zur Absättigung unspezifischer Bindungsstellen an die Flächenelemente gebunden. Das verhindert ein unspezifisches Binden der dritten und/oder vierten Nukleinsäure an den Untergrund im Bereich der Flächenelemente. Es ist nicht erforderlich, die unspezifischen Bindungsstellen auf den Flächenelementen unmittelbar vor der Identifizierung der Markierung zu blockieren. Das Verfahren ist unter anderem dadurch sehr schnell.

Nach einer Ausgestaltung sind die erste bzw. zweite Nukleinsäure über hydrophile Linker an den einen bzw. anderen Teil der Flächenelemente gebunden. Die hydrophilen Linker können aus der folgenden Gruppe ausgewählt sein: Peptide, Polyethylenglykole, polymere Zucker, Polyacrylamid, Polyimine, Dendrimermoleküle. Das Vorsehen solcher Linker verbessert die Zugänglichkeit der Nukleinsäuren für die dritten und ggf. die vierten Nukleinsäure. Zweckmäßig ist es außerdem, daß der hydrophile Linker in einem Abschnitt an die erste bzw. dritte Nukleinsäure gebunden ist welcher nicht komplementär zu der zweiten bzw. vierten Nukleinsäure ist. Das gewährleistet, eine Hybridisierung der zueinander komplementären Nukleinsäuren. Der Linker kann vorteilhafter Weise auch endständig an die erste bzw. dritte Nukleinsäure gebunden sein. Die Empfindlichkeit des Verfahrens wird gesteigert. Weiterhin kann zumindest eine der Nukleinsäuren mittels Partikel, insbesondere Agarose-Partikel, an die Flächenelemente gebunden werden. Das ist besonders dann vorteilhaft, wenn es die Oberfläche des festen Körpers nicht zuläßt, daß die Nukleinsäuren oder Linker direkt daran gebunden werden.

Besonders vorteilhaft ist es, zumindest eine der Nukleinsäuren mittels einer Drucktechnik, insbesondere Inkjet-Technik, auf die Flächenelemente aufzubringen. Ein solches Verfahren ermöglicht es, in einem Produktionsablauf unterschiedliche Markierungen in großer Anzahl automatisiert auf festen Körpern, z.B. Verpackungen, anzubringen.

Nach einer weiteren Ausgestaltung werden die ersten und/oder zweiten Nukleinsäuren an einer vorgegebenen Stelle ihrer Struktur an die Flächenelemente gebunden. Durch diese Maßnahme kann verhindert werden, daß die ersten und/oder zweiten Nukleinsäuren an deren Bindungsstellen für die dritten bzw. vierten Nukleinsäuren an die Flächenelemente binden.

Das Teilmuster kann in Form eines Bar-Codes vorliegen. Vorteilhafterweise ist das Teilmuster in Form eines Rasters ausgebildet. Die Flächenelemente können rund, vorzugsweise mit einen Durchmesser kleiner als 100 µm, ausgebildet sein.

Erfindungsgemäß erfolgt der Nachweis der Bindung durch veränderte optische Eigenschaften der gebundenen Nukleinsäuren. Eine optische Eigenschaft ist z.B. das Absorptionsvermögen für Licht bestimmter Wellenlängen. Die Änderung des Absorptionsvermögens durch die Bindung kann zu einer Farbänderung führen. Der Nachweis durch veränderte Eigenschaften erfordert keine weitere chemische oder biochemische Nachweisreaktion. Ein Extrahieren oder Entfernen der gebundenen Nukleinsäuren von dem festen Körper ist nicht erforderlich. Die Identifizierung erfolgt einfach und schnell.

Zumindest eines der Nukleinsäuren muß ein Fluorophor aufweisen, das beim Binden seine Fluoreszenzeigenschaften ändert. Eine solche Nukleinsäure kann beispielsweise in Form eines in der EP 0 745 690 A2 offenbarten sogenannten "molecular beacons" ausgebildet sein. Die Bindung eines solchen Moleküls an eine entsprechende komplementäre Nukleinsäure führt zu einer deutlichen Verstärkung seiner Fluoreszenz. Die Fluoreszenz kann unmittelbar nach der Bindung detektiert werden. Bei geeigneter Wahl der Markierungssubstanz sind gebundene Nukleinsäuren mit bloßem Auge erkennbar.

Erfindungsgemäß werden die dritten und/oder vierten Nukleinsäuren durch Auftropfen, Aufsaugen, Sprühen oder Zerstäuben homogen verteilt mit der Markierung in Kontakt gebracht. Ein solches Verfahren hat den Vorteil, sehr einfach handhabbar zu sein. Die dritten und/oder vierten Nukleinsäuren können in Lösung, z.B. aus einer Spraydose, auf die Markierung aufgesprüht werden. Kurze Zeit später können spezifisch gebundene Nukleinsäuren detektiert werden.

Bei dem einstufigen Detektionsverfahren handelt es sich zweckmäßigerweise um ein Verfahren, welches ohne Waschschritte durchgeführt wird. Das einstufige Detektionsverfahren kann ferner unter Ausnutzung einer der folgenden Effekte durchgeführt werden: Bildung oder Trennung eines Donor/Akzeptorpaars, Oberflächen-Plasmonen-Resonanz, Gewichtsunterschied, Einlagerung oder Freisetzung von Interkalatoren. Vorteilhaft ist insbesondere die Ausnutzung der Bildung oder Trennung eines Donor/Akzeptorpaars. Ein solcher Effekt tritt beispielsweise bei Verwendung von "molekular beacons" auf.

Die Markierung enthält die erste Nukleinsäure vorteilhafterweise in einer Menge von höchstens 10 µg Das Verfahren benötigt äußerst geringe Mengen an Nukleinsäuren.

Zur Lösung der erfindungsgemäßen Aufgabe ist ferner vorgesehen ein Träger zur Befestigung auf einen festen Körper, wobei auf der einen Seite des Trägers eine aus Flächenelementen gebildete vorgegebene Markierung aufgebracht ist, wobei an einem ersten Teil der Flächenelemente erste Nukleinsäuren gebunden sind, so daß ein erstes Teilmuster gebildet ist, und wobei der Träger als Folie ausgebildet ist, die auf der einen Seite mit Klebstoff beschichtet ist. - Ein solcher Träger kann einfach auf zu markierenden festen Körpern befestigt werden.

Nach einer weiteren Ausgestaltung sind an einen zweiten Teil der Flächenelemente zweite Nukleinsäuren gebunden, so daß ein zweites Teilmuster gebildet ist. Das ermöglicht eine besonders sichere Identifizierung des ersten Teilmusters.

Die eine Seite, d.h. die mit Biomolekülen beschichtete Seite, kann mit einer abziehbaren Schutzfolie abgedeckt sein. Gleichfalls kann die Klebstoffschicht mit einer abziehbaren weiteren Schutzfolie abgedeckt sein.

Nach weiterer Maßgabe der Erfindung ist ein Kit mit einem erfindungsgemäßen Träger und mit einer dritten zur ersten Nukleinsäure affinen Nukleinsäure vorgesehen. Diese Nukleinsäure kann in Lösung vorliegen. Ferner kann im Kit eine vierte zur zweiten Nukleinsäure affine Nukleinsäure enthalten sein.

Zur Herstellung des Trägers kommen alle geeigneten Materialien in Betracht. Besonders bevorzugt sind aus Kunststoff oder Metall hergestellte Folien.

Die genannten und die nachstehend noch zu erläuternden Merkmale sind nicht nur in den jeweils angegebenen Kombinationen, sondern auch in anderen Kombinationen oder in Alleinstellung verwendbar. Weitere vorteile ergeben sich aus folgenden Ausführungsbeispielen und im Zusammenhang mit den Zeichnungen. Hierin zeigen:
- Fig. 1 a, b, c: eine schematische Darstellung von Flächenele- menten mit daran gebundenen Nukleinsäuren,
- Fig. 2 a, b: eine schematische Darstellung der Identifi- zierung eines Flächenelements mit daran ge- bundenen Nukleinsäuren durch molecular beacons,
- Fig. 3: eine schematische Darstellung der Identifi- zierung einer auf einem festen Körper aufge- brachten Markierung,
- Fig. 4: ein aus Partikeln bestehendes erstes Teilmu- ster eines ersten Ausführungsbeispiels im Durchlicht,
- Fig. 5: das Teilmuster gemäß Fig. 4 zusammen mit ei- nem aus weiteren Partikeln gebildeten zweiten Teilmuster im Durchlicht und
- Fig. 6: die Teilmuster nach Fig. 5 bei UV-Anregung.
- Fig. 7: ein zweites Ausführungsbeispiel,
- Fig. 8: eine vergrößerte Darstellung nach Fig. 7,
- Fig. 9: ein drittes Ausführungsbeispiel, hergestellt mit einer Konzentration von 0,5 pmol/µl,
- Fig. 10: das Ausführungsbeispiel nach Fig. 9, herge- stellt mit einer Konzentration von 1,0 pmol/µl,
- Fig. 11: das Ausführungsbeispiel nach Fig. 9, herge- stellt mit einer Konzentration von 2,0 pmol/µl und
- Fig. 12: das Ausführungsbeispiel nach Fig. 9, herge- stellt mit einer Inkubationszeit von 6 Stun- den (Konzentration 2,0 pmol/µl).

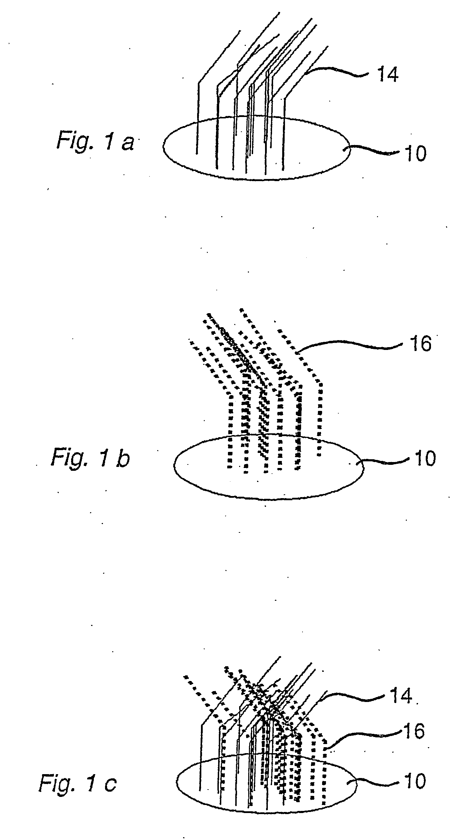

Fig. 1 a zeigt ein Flächenelement 10 mit daran gebundenen ersten Nukleinsäuren 14. In Fig. 1 b ist ein Flächenelement 10 mit daran gebundenen zweiten Nukleinsäuren 16 dargestellt. Fig. 1 c zeigt ein Flächenelement 10 mit daran gebundenen ersten 14 und zweiten Nukleinsäuren 16.

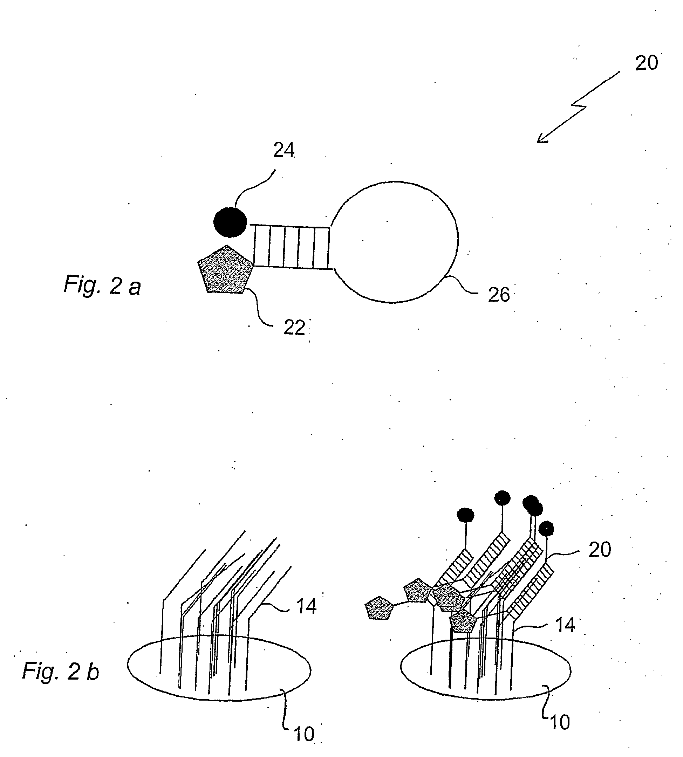

In Fig. 2 a ist ein molecular beacon 20 schematisch dargestellt. Dabei handelt es sich um ein haarnadelförmiges DNA-Molekül. Der DNA-Strang dieses DNA-Moleküls weist an seinen Enden zueinander komplementäre Bereiche auf. Diese Bereiche liegen basengepaart vor. Am einen Ende des DNA-Strangs befindet sich ein Fluorophor 22, wie Fluorescein, am anderen Ende ein Quencher 24, wie 4-Dimethylamino-azobenzol-4'-sulfonyl-chlorid. Bei Bestrahlung des molecular beacons 20 mit Licht einer Anregungswellenlänge des Fluorophors 22 erfolgt keine Emission von Licht. Statt dessen kommt es zu einem strahlungslosen Energieübergang auf den Quencher 24. In der Schlaufe 26 des molecular beacons 20 befindet sich eine (hier nicht gezeigte) Nukleotidsequenz, die komplementär zu einer Nukleotidsequenz der ersten Nukleinsäure 14 ist.

Fig. 2 b zeigt links eine schematische Darstellung eines Flächenelements 10 mit daran gebundenen ersten Nukleinsäuren 14. Rechts ist dieses Flächenelement 10 nach der Bindung von molecular beacons 20 dargestellt. Die molecular beacons 20 binden mit den Nukleotidsequenzen in den Schlaufen 26 an die komplementären Nukleotidsequenzen der ersten Nukleinsäuren 14. Dies führt zu dem Lösen der Basenpaarungen im Bereich der Enden der DNA-Stränge der molecular beacons 20. Durch die Bindung werden die Fluorophore 22 von den Quenchern 24 räumlich getrennt. Ein strahlungsloser Energieübergang von den Fluorophoren 22 auf die Quencher 24 kann nicht mehr erfolgen. Bei Anregung der Fluorophore 22 mit Licht einer Anregungswellenlänge erfolgt eine meßbare oder sogar mit dem bloßem Auge sichtbare Emission von Licht.

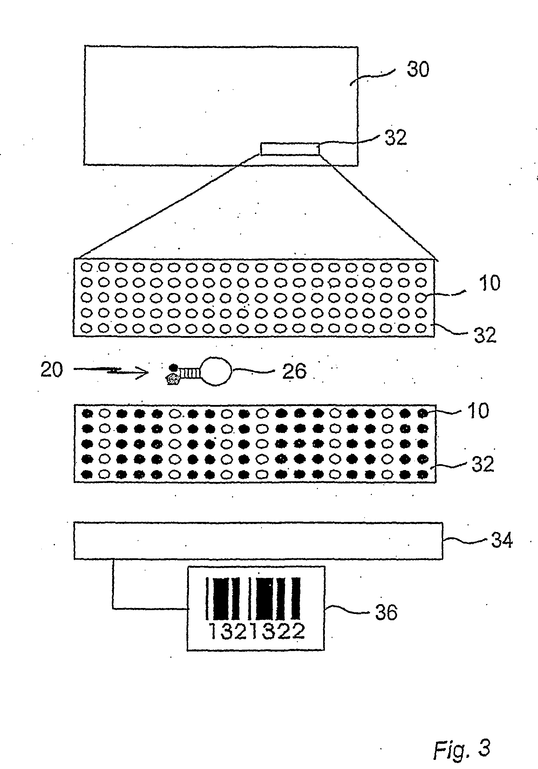

Fig. 3 zeigt einen festen Körper 30, wie z.B. eine Banknote, mit einer Markierung 32. Die Markierung 32 besteht aus einem Raster von Flächenelementen 10. An den einen Teil der Flächenelemente 10 sind hier nicht dargestellte erste Nukleinsäuren 14 gebunden. Diese weisen je eine Nukleotidsequenz auf, die zu der Nukleotidsequenz der Schlaufe 26 eines molecular beacons 20 komplementär ist. An den anderen Teil der Flächenelemente sind dazu nicht komplementäre zweite Nukleinsäuren 16 gebunden, die hier ebenfalls nicht gezeigt sind. Zur Absättigung unspezifischer Bindungsstellen ist außerdem eine weitere Nukleinsäure an die Flächenelemente 10 gebunden. Die molecular beacons 20 sind in einer Lösung enthalten. Die Markierung 32 wird mit dieser Lösung in Kontakt gebracht. Um stringente Bindungsbedingungen zu gewährleisten, weist die Lösung eine definierte Ionenstärke auf und das Inkontaktbringen erfolgt bei einer erhöhten Temperatur. Unter diesen Bedingungen binden die molecular beacons 20 über die erste Nukleinsäure 14 nur an den einen Teil der Flächenelemente 10. Sie binden nicht unspezifisch an die Flächenelemente 10, da unspezifische Bindungsstellen abgesättigt worden sind. Sie binden auch nicht an die zum Absättigen verwendeten weiteren Nukleinsäuren und an die zweiten Nukleinsäuren 16 an dem anderen Teil der Flächenelemente 10.

Würde die Stringenz der Bindungsbedingungen verringert werden, könnten auch unspezifische molecular beacons an die ersten 14 bzw. zweiten Nukleinsäuren 16 binden. In diesem Fall ist eine Identifizierung des Teilmusters nicht möglich.

Flächenelemente 10 mit gebundenen molecular beacons 20 sind als schwarz ausgefüllte Kreisflächen, die anderen als nicht ausgefüllte Kreisflächen dargestellt. Wird die Markierung 32 mit Licht einer geeigneten Wellenlänge bestrahlt, so fluoreszieren die gebundenen molecular beacons 20. Ein Detektor 34 mißt und lokalisiert die Fluoreszenz. Er stellt auf einem Ausgabegerät 36 das erzeugte Teilmuster dar. Die Sicherheit des Verfahrens kann gesteigert werden, indem die an den anderen Teil der Flächenelemente gebundenen zweiten Nukleinsäuren 16 zusätzlich mit spezifischen, hier nicht dargestellten weiteren molecular beacons nachgewiesen werden. Die weiteren molecular beacons weisen gegenüber den molecular beacons 20 ein anderes Fluorophor mit deutlich anderer Fluoreszenz auf. Dadurch lassen sich sowohl spezifisch gebundene molecular beacons 20 als auch spezifisch gebundene weitere molecular beacons nachweisen. Der Kontrast zwischen dem einen und dem anderen Teil der Flächenelemente ist gegenüber dem Kontrast beim bloßen Einsatz der molecular beacons 20 deutlich erhöht. Der verbesserte Kontrast erhöht die Sicherheit beim Ablesen der Fluoreszenz. Das ermöglicht eine Identifizierung von kleinen oder schmalen Teilmustern.

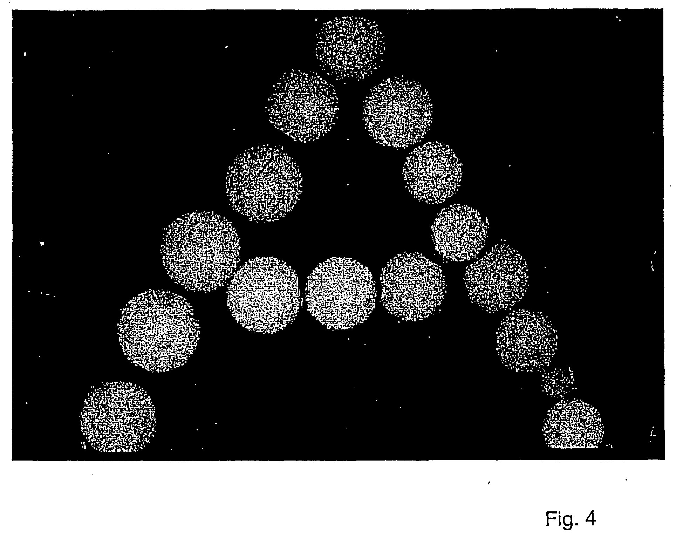

Die in den Fig. 4 bis 6 gezeigte Markierung ist wie folgt hergestellt worden:
Zunächst werden aus Glas hergestellte Objektträger nacheinander jeweils 30 Minuten in Wasser, 6 % Ammoniak, 5 % H₂O₂, Wasser, Azeton und 2 % 3-Aminopropyltriethoxsilan in Aceton, und danach in Aceton inkubiert. Anschließend werden die derart vorbehandelten Objektträger bei 37° C für eine Stunde getrocknet.
Zur Herstellung der Markierung werden, vorzugsweise kreuzvernetzte 4 %, Aldehyd-aktiviert Partikel mit einem durchschnittlichen Durchmesser von 80 µm verwendet. Die Partikel werden in PBS (Phosphat-gepufferte Saline, 10 mM Natriumphosphat, 150 mM NaCl, pH 7,4) durch Suspendieren und Zentrifugieren gewaschen und in einem Volumenverhältnis von 1 : 1 suspendiert. Zu 50 µl Partikel-Suspension werden 5 µl 20 µM Amino-aktiviertes Oligonukleotid gelöst in Wasser zugeführt.
Die Partikel-Suspension wird zusammen mit dem Oligonukleotid für eine Stunde bei Raumtemperatur unter leichtem Schütteln inkubiert. Als Oligonukleotid kann z.B. ein Oligonukleotid folgender Sequenz benutzt werden:
   5'-Amino-TCCAAGCCTGGAGGGATGATACTTTGCGCTTGG-3'
Anschließend wird auf einen Amino-aktivierten Objektträger eine Kunststoffschablone gelegt, welche eine Aussparung in Form des Buchstabens "A" aufweist. Die hergestellte mit Oligonukleotiden versetzte Partikel-Suspension wird durch Zugabe von 1 M Natriumcyanoborhydrid (Firma Sigma, München) in 10 mM NaOH gelöst und auf 50 mM Natriumcyanoborhydrid gebracht. Die Partikel-Suspension wird dann auf die Schablone aufgetragen. Durch die Aussparungen in der Schablone kommt die Partikel-Suspension mit der Amino-aktivierten Oberfläche des Objektträgers in Kontakt. Die Partikel-Suspension ist im Kontakt mit der Oberfläche des Objektträgers für etwa 20 Stunden bei Raumtemperatur in einer feuchten Kammer inkubiert worden.
Anschließend werden Aldehyd-aktivierte Partikel in PBS durch Suspendieren und Zentrifugieren gewaschen und in einem Volumenverhältnis von 1 : 1 suspendiert. Zu 2 µl Partikel-Suspension werden 20 µl 20 µM Amino-aktiviertes weiteres Oligonukleotid gelöst in Wasser zugefügt. Die Partikel-Suspension und das weitere Oligonukleotid werden eine Stunde bei Raumtemperatur unter leichtem Schütteln inkubiert. Als weiteres Oligonukleotid ist ein Oligonukleotid der folgenden Sequenz verwendet worden:
   5'-Amino-TTGGAATCCATGGTTAAACTTGTACTTTAGGTC-3'

Die mit dem weiteren Oligonukleotid beschichteten Partikel sind nach Entfernen der Schablone auf dem Objektträger aufgebracht worden. Überschüssige Partikel sind durch Absaugen mit einer Glaskapillare unter dem Mikroskop entfernt worden. Es ist ein Kunststoffrahmen auf dem Objektträger aufgelegt worden. Die Partikel-Suspension mit dem weiteren Oligonukleotid ist durch Zugabe von 1 M Natriumcyanoborhydrid gelöst in 10 mM NaOH auf 50 mM Natriumcyanoborhydrid gebracht worden. Es ist Partikel-Suspension mit weiterem Oligonukleotid innerhalb des Rahmens aufgetragen und über Nacht bei Raumtemperatur in einer feuchten Kammer inkubiert worden. Zur Entfernung nicht gebundener Partikel ist der Objektträger mehrmals in TE (10mM TrisCl, 1 mM EDTA, pH 8) gewaschen und in einer feuchten Kammer in TE mit 0,05 % Natriumazid gelagert worden.

Zur Identifizierung der durch das Oligonukleotid 1 hergestellten Markierung ist ein "molecular beacon" der folgenden Sequenz in einer Konzentration von 50 nM in TE gelöst auf dem Objektträger aufgebracht worden:
3'-X-GGTTCGGACCTCCCTACTATGAAACGCGAACC-6FAM-5';X = dt(C2-DABCYL).

Die Sequenz des "molecular beacon" ist komplementär der Sequenz des Oligonukleotids. Die Lösung ist auf dem Objektträger mit einer Temperatur von 37° C mittels eines Zerstäubers aufgetragen worden. Der Objektträger ist vor und nach der Zugabe der Lösung mit Licht einer Wellenlänge von 496 nm bestrahlt worden. Die Emission bei einer Wellenlänge von 516 nm ist 5 Minuten nach der Zugabe der Lösung gemessen worden.

Fig. 4 zeigt Partikel mit daran kovalent gebundenem Oligonukleotid auf einer Amino-beschichteten Oberfläche eines Objektträgers. Die Partikel sind in Form des Buchstabens "A" aufgebracht worden. Sie bilden ein erstes Teilmuster.

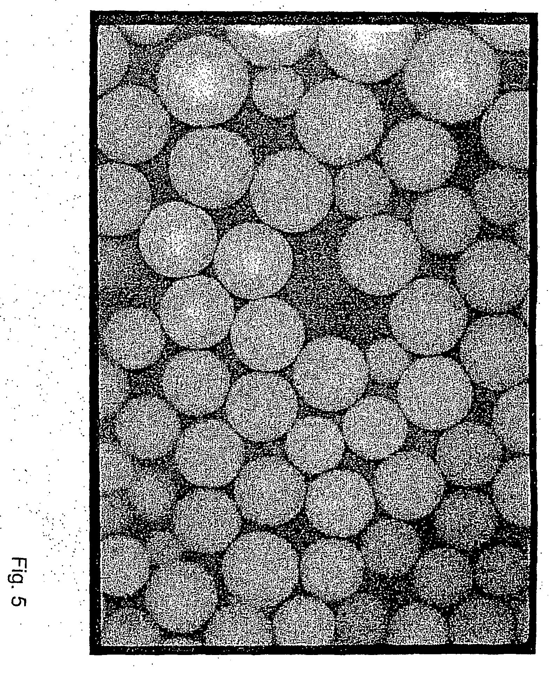

Fig. 5 zeigt das erste Teilmuster gemäß Fig. 4 in Kombination mit einem zweiten Teilmuster. Das zweite Teilmuster ist aus Partikeln gebildet, welche mit kovalent gebundenem weiterem Oligonukleotid beschichtet sind. Das erste und das zweite Teilmuster können im Durchlicht voneinander nicht unterschieden werden.

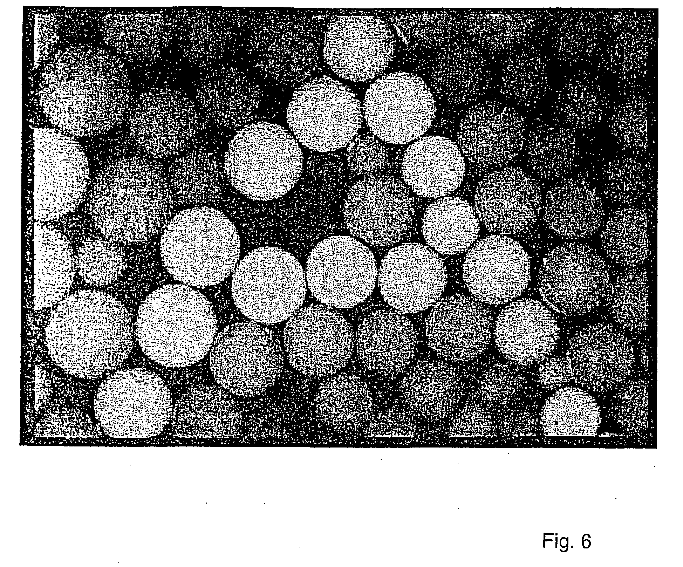

Fig. 6 zeigt die Teilmuster gemäß Fig. 5 nach Inkubation mit dem "molecular beacon", welches komplementär zum Oligonukleotid ist. Durch die Hybridisierung des "molecular beacons" mit dem Oligonukleotid ist bei Anregung mit UV-Licht schon nach wenigen Minuten eine Fluoreszenz beobachtbar. Das erste Teilmuster ist identifizierbar. Es ist deutlich in Form des Buchstabens "A" erkennbar.

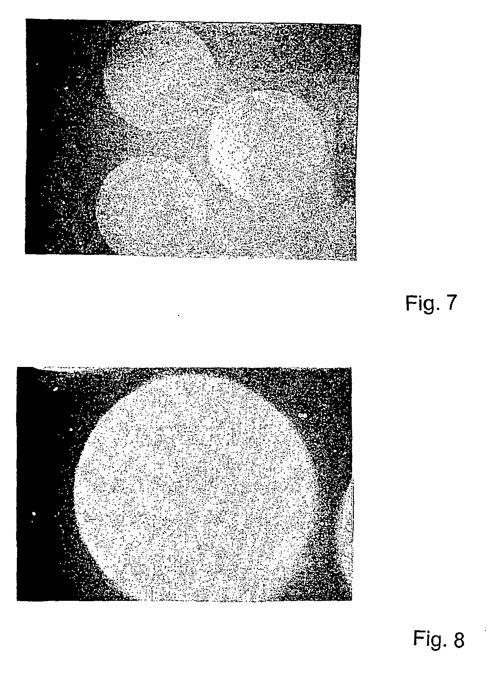

Bei dem in Fig. 7 gezeigten zweiten Ausführungsbeispiel wurde als Träger eine Polycarbonatfolie der Stärke 0,25 mm verwendet. Diese wurde nach Reinigung mit Isopropanol in einem ersten Schritt mittels 5N NaOH für 30 min aktiviert und danach mit H₂O gewaschen.

Anschließend erfolgte in einem zweiten Schritt die direkte Kopplung der Biopolymere an den Träger. Als Biopolymer wurden aminomodifizierte DNA-Oligomere einer Sequenz N, welche z.B. die vorbeschriebene Sequenz sein kann, verwendet.

Dazu wurde eine Lösung aus 5 µl DNA-Oligomer (100 µM), 1 µl 1-Ethyl-3-(3-dimethylaminopropyl)carbodiimid (EDC) und 4 µl 0,1 M Carbonatpuffer, pH 9,5 angesetzt. Jeweils 1 µl dieser Lösung wurde auf dem voraktivierten Träger punktweise aufgebracht, so daß sich ein charakteristisches Muster ergab.

Zur vollständigen Anbindung wurde über Nacht in wassergesättigter Atmosphäre inkubiert und danach überschüssige DNA-Oligomere durch Waschen mit H₂O und 0,1% Tween 20 entfernt.

Die Detektion der Bindung erfolgte mit einem weiteren DNA-Oligomer in Form eines molecular beacons mit der Sequenz N', welche teilweise komplementär der Sequenz N war. Diese wurden in einer Konzentration von 1,0 pmol/µl auf die Markierung aufgebracht und nach ca. 30 s im Fluoreszenzmikroskop vermessen.

Fig. 7 zeigt eine Teilmuster aus einer direkt am Träger gekoppelten DNA-Markierung. In Fig. 8 ist ein Punkt dieser Markierung gesondert herausgestellt.

Bei einem dritten Ausführungsbeispiel wurde eine Polycarbonatfolie der Stärke 0,25mm mit einer Mischung aus 100 Teilen Ethanol und 1 Teil Glycidylsilan für 30 Minuten inkubiert. Dabei wird auf der Oberfläche Silan abgeschieden. Überschüssiges Silan wurde mit Wasser vom Träger abgewaschen und der Träger im Stickstoff-Strom trocken geblasen. Anschließend wurde das Silan auf dem Träger bei 80°C für 60 Minuten vernetzt.

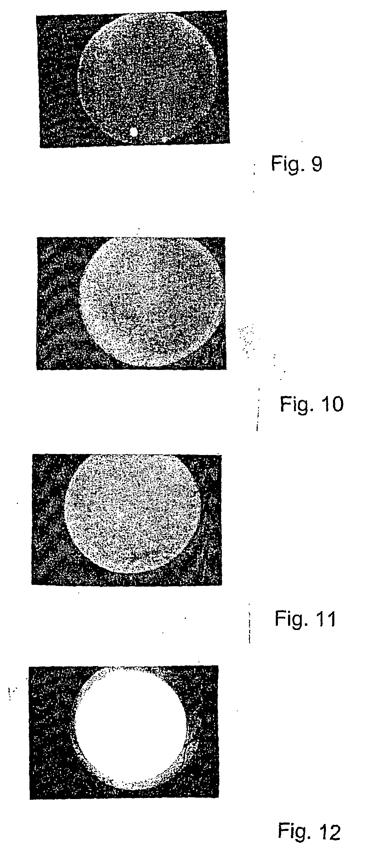

Aminomodifizierte Oligonukleotide einer Sequenz N, die z.B. die vorgeschriebene Sequenz sein kann, werden im Carbonatpuffer; 0,1M; pH 9,5; auf Konzentrationen von 0,5 pmol/µl, 1,0 pmol/µl und 2,0 pmol/µl verdünnt und als 1µl große Tropfen auf die aktivierten Träger aufgebracht und für 30 Minuten inkubiert (Fig. 9 bis 11). Bei der in Fig. 12 gezeigten Probe hat die Inkubation 6 Stunden gedauert.

Die mit DNA beschichteten Träger werden mit einem molecular beacon der Seqenz N', welche teilweise komplementär zur Sequenz N ist, der Konzentration 1 pmol je µl für ca. 30 sec inkubiert und danach im Fluoreszenzmikroskop gemessen.

Das Ergebnis ist aus den Fig. 9 bis 12 ersichtlich:
Je höher die Konzentration an Oligonukleotiden in dem Tropfen ist, desto höher ist die Belegungsdichte und desto heller erscheint die hergestellte Markierung. Es lassen sich durch eine Variation der Konzentration der Oligonukleotide also auch Markierungen mit unterschiedlichen Intensität herstellen.

### Bezugszeichenliste

- 10: Flächenelement,
- 14: erste Nukleinsäure,
- 16: zweite Nukleinsäure,
- 20: molecular beacon,
- 22: Fluorophor,
- 24: Quencher,
- 26: Schlaufe,
- 30: fester Körper,
- 32: Markierung,
- 34: Detektor
- 36: Ausgabegerät

## Patentansprüche

1. Verfahren zur Identifizierung eines markierten Produkts, wobei das Produkt ein fester Körper ist und wobei eine auf den festen Körper (30) aufgebrachte aus Flächenelementen (10) gebildete vorgegebene Markierung (32), bei der an einen ersten Teil der Flächenelemente (10) erste Nukleinsäuren gebunden sind, so daß ein erstes vorgegebenes Teilmuster gebildet ist, mit folgenden Schritten identifiziert wird:
a) Inkontaktbringen der Markierung (32) mit in einer Lösung enthaltenen, zu den ersten Nukleinsäuren affinen dritten Nukleinsäuren, so daß die ersten und die dritten Nukleinsäuren hybridisieren, wobei die dritten Nukleinsäuren durch Auftropfen, Aufsaugen, Sprühen oder Zerstäuben homogen verteilt mit der Markierung (32) in Kontakt gebracht werden, und
b) Identifizierung des durch die hybridisierten ersten und dritten Nukleinsäuren gebildeten ersten Teilmusters auf dem festen Körper durch Nachweis der Hybridisierung zwischen den ersten und den dritten Nukleinsäuren mittels eines einstufigen Detektionsverfahrens, indem eine Änderung einer Fluoreszenzeigenschaft eines an der ersten oder dritten Nukleinsäure gebunden Fluorophors ohne Waschschritte detektiert wird
wobei die Markierung direkt auf dem markierten Produkt identifiziert wird, ohne daß sie davon gelöst wird.

2. Verfahren nach Anspruch 1, wobei vor dem Schritt lit. b) der folgende Schritt durchgeführt wird: Binden zweiter Nukleinsäuren an einen zweiten Teil der Flächenelemente (10), so daß ein zweites Teilmuster gebildet ist.

3. Verfahren nach Anspruch 2, wobei beim Schritt lit. b) zusätzlich vierte, zu den zweiten Nukleinsäuren affine Nukleinsäure mit der Markierung (32) in Kontakt gebracht werden, und wobei beim Schritt lit. b) die Hybridisierungen zwischen den zweiten und den vierten Nukleinsäuren nachgewiesen und das durch die gebundenen zweiten und vierten Nukleinsäuren gebildete Teilmuster identifiziert wird.

4. Verfahren nach Anspruch 3, wobei die vierten Nukleinsäuren in einer Lösung enthalten sind.

5. Verfahren nach einem der vorhergehenden Ansprüche, wobei das Inkontaktbringen unter vorgegebenen stringenten Bindungsbedingungen, vorzugsweise bei Raumtemperatur, durchgeführt wird.

6. Verfahren nach einem der vorhergehenden Ansprüche, wobei mindestens ein weiteres Biopolymer oder die zweite Nukleinsäure zur Absättigung unspezifischer Bindungsstellen an die Flächenelemente (10) gebunden wird.

7. Verfahren nach einem der vorhergehenden Ansprüche, wobei die erste bzw. zweite Nukleinsäure über hydrophile Linker an den einen bzw. anderen Teil der Flächenelemente gebunden wird.

8. Verfahren nach einem der vorhergehenden Ansprüche, wobei die hydrophilen Linker aus der folgenden Gruppe ausgewählt sind: Peptide, Polyethylenglykole, polymere Zucker, Polyacrylamid, Polyimine oder Dendrimermoleküle.

9. Verfahren nach einem der vorhergehenden Ansprüche, wobei der hydrophile Linker in einem Abschnitt an die erste bzw. dritte Nukleinsäure gebunden ist, welcher nicht komplementär zu der zweiten bzw. vierten Nukleinsäure ist.

10. Verfahren nach einem der vorhergehenden Ansprüche, wobei zumindest eine der Nukleinsäuren mittels Partikel, insbesondere Agarose-Partikel, an die Flächenelemente (10) gebunden wird.

11. Verfahren nach einem der vorhergehenden Ansprüche, wobei zumindest eine der Nukleinsäuren mittels einer Drucktechnik, insbesondere Inkjet-Technik, auf die Flächenelemente (10) aufgebracht wird.

12. Verfahren nach einem der vorhergehenden Ansprüche, wobei die ersten und/oder zweiten Nukleinsäuren an einer vorgegebenen Stelle ihrer Struktur an die Flächenelemente (10) gebunden werden.

13. Verfahren nach einem der vorhergehenden Ansprüche, wobei das Teilmuster in Form eines Bar-Codes vorliegt.

14. Verfahren nach einem der vorhergehenden Ansprüche, wobei das Teilmuster in Form eines Rasters ausgebildet ist.

15. Verfahren nach einem der vorhergehenden Ansprüche, wobei Flächenelemente (10) rund, vorzugsweise mit einen Durchmesser kleiner als 100 µm, ausgebildet sind.

16. Verfahren nach einem der Ansprüche 3 bis 15, wobei die vierten Nukleinsäuren durch Auftropfen, Aufsaugen, Sprühen oder Zerstäuben homogen verteilt mit der Markierung (32) in Kontakt gebracht werden.

17. Verfahren nach einem der vorhergehenden Ansprüche, wobei das einstufige Detektionsverfahren unter Ausnutzung des folgenden Effekts durchgeführt wird:
Bildung oder Trennung eines Donor/Akzeptor-Paars.

18. Verfahren nach einem der vorhergehenden Ansprüche, wobei die Markierung die erste Nukleinsäure in einer Menge von höchstens 10 µg enthält.

## Claims

1. A method for identifying a marked product, wherein said product is a solid body (30) and wherein a predetermined mark (32) applied to said solid body and formed from area elements (10), in which first nucleic acids are bound to a first part of the area elements (10) so that a first predetermined part-pattern is formed, is identified by the following steps:
a) contacting the mark (32) with third nucleic acids present in a solution and having affinity for the first nucleic acids so that the first and the third nucleic acids hybridize, wherein the third nucleic acids are contacted with the mark (32) homogeneously distributed by dropwise application, absorption, spraying or atomization; and
b) identifying the first part-pattern formed by the hybridized first and third nucleic acids on the solid body by detecting hybridization of the first and third nucleic acids with a one-stage detection method, in which a change in a fluorescent characteristic of a fluorophor that is bound to the first or third nucleic acid is detected without washing steps;
wherein the mark is directly identified on the marked product without being detached from it.

2. A method according to claim 1, wherein the following step is carried out before step b): binding of second nucleic acids to a second part of the area elements (10) so that a second part-pattern is formed.

3. A method according to claim 2, wherein step b) comprises contacting additional fourth nucleic acids having affinity for the second nucleic acids with the mark (32), and wherein in step b) the hybridization between the second and fourth nucleic acids is detected and the part-pattern formed by the bound second and fourth nucleic acids is identified.

4. A method according to claim 3, wherein the fourth nucleic acids are present in a solution.

5. A method according to any of the preceding claims, wherein the contacting is carried out under predetermined stringent binding conditions, preferably at room temperature.

6. A method according to any of the preceding claims, wherein at least one other biopolymer or the second nucleic acid is bound to the area elements (10) to saturate nonspecific binding sites.

7. A method according to any of the preceding claims, wherein the first and second nucleic acid are bound via hydrophilic linkers respectively to the one or other part of the area elements (10).

8. A method according to any of the preceding claims, wherein the hydrophilic linkers are selected from the following group: peptides, polyethylene glycols, polymeric sugars, polyacrylamide, polyimines, or dendrimer molecules.

9. A method according to any of the preceding claims, wherein the hydrophilic linker is bound to the first or third nucleic acid in a section which is not complementary respectively to the second or fourth nucleic acid.

10. A method according to any of the preceding claims, wherein at least one of the nucleic acids is bound to the area elements (10) via particles, in particular agarose particles.

11. A method according to any of the preceding claims, wherein at least one of the nucleic acids is applied to the area elements (10) by means of a printing technique, in particular inkjet technique.

12. A method according to any of the preceding claims, wherein the first and/or second nucleic acids are bound to the area elements (10) at a predetermined site of their structure.

13. A method according to any of the preceding claims, wherein the part-pattern is in the form of a barcode.

14. A method according to any of the preceding claims, wherein the part-pattern is formed as an array.

15. A method according to any of the preceding claims, wherein the area elements (10) are round, preferably with a diameter of less than 100 µm.

16. A method according to any of claims 3 to 15, wherein the fourth nucleic acids are contacted with the mark (32) homogeneously distributed by dropwise application, absorption, spraying or atomization.

17. A method according to any of the preceding claims, wherein the one-stage detection method is carried out by utilizing the following effect: formation or separation of a donor/acceptor pair.

18. A method according to any of the preceding claims, wherein the mark contains the first nucleic acid in an amount not exceeding 10 µg.

## Revendications

1. Procédé d'identification d'un produit marqué, dans lequel le produit est un corps solide et dans lequel un marquage (32) prédéfini, appliqué sur le corps solide (30) et formé d'éléments plans (10), sur une première partie desquels éléments plans (10) des premiers acides nucléiques sont liés de manière à former un premier modèle partiel prédéfini, est identifié avec les étapes suivantes :
a) mise en contact du marquage (32) avec des troisièmes acides nucléiques affines des premiers acides nucléiques contenus dans une solution de sorte que les premiers et les troisièmes acides nucléiques s'hybrident, les troisièmes acides nucléiques étant mis en contact avec le marquage (32) par distribution homogène par addition par goutte, aspiration, pulvérisation ou atomisation, et
b) identification du premier modèle partiel formé par les premiers et troisièmes acides nucléiques hybridés sur le corps solide en décelant l'hybridation entre les premiers et les troisièmes acides nucléiques au moyen d'un procédé de détection en une étape, dans lequel une variation d'une propriété de fluorescence d'un fluorophore lié au premier ou au troisième acide nucléique est détectée sans étapes de lavage,
dans lequel le marquage est identifié directement sur le produit marqué sans en être détaché.

2. Procédé selon la revendication 1, dans lequel, avant l'étape b), on réalise l'étape suivante consistant à lier des seconds acides nucléiques sur une seconde partie des éléments plans (10) de manière à former un second modèle partiel.

3. Procédé selon la revendication 2, dans lequel, lors de l'étape b), on met en outre en contact avec le marquage (32) des quatrièmes acides nucléiques affines des deuxièmes acides nucléiques et dans lequel, à l'étape b), on décèle les hybridations entre les deuxièmes et les quatrièmes acides nucléiques et on identifie le modèle partiel formé par les deuxièmes et quatrièmes acides nucléiques liés.

4. Procédé selon la revendication 3, dans lequel les quatrièmes acides nucléiques sont contenus dans une solution.

5. Procédé selon l'une quelconque des revendications précédentes, dans lequel la mise en contact s'effectue dans des conditions de liaison stringentes prédéfinies, de préférence à température ambiante.

6. Procédé selon l'une quelconque des revendications précédentes, dans lequel au moins un autre biopolymère ou le deuxième acide nucléique est lié aux éléments plans (10) pour saturer des points de liaison non spécifiques.

7. Procédé selon l'une quelconque des revendications précédentes, dans lequel le premier ou le deuxième acide nucléique est lié par des lieurs hydrophiles à l'une ou l'autre partie des éléments plans.

8. Procédé selon l'une quelconque des revendications précédentes, dans lequel les lieurs hydrophiles sont choisis parmi le groupe suivant : peptides, polyéthylèneglycols, sucres polymères, polyacrylamide, polyimines ou molécules dendrimères.

9. Procédé selon l'une quelconque des revendications précédentes, dans lequel le lieur hydrophile est lié dans un segment au premier ou au troisième acide nucléique, qui n'est pas complémentaire du deuxième ou du quatrième acide nucléique.

10. Procédé selon l'une quelconque des revendications précédentes, dans lequel au moins l'un des acides nucléiques est lié aux éléments plans (10) au moyen de particules, en particulier de particules d'agarose.

11. Procédé selon l'une quelconque des revendications précédentes, dans lequel au moins l'un des acides nucléiques est appliqué sur les éléments plans (10) au moyen d'une technique d'impression, en particulier d'une technique à jet d'encre.

12. Procédé selon l'une quelconque des revendications précédentes, dans lequel les premiers et/ou les deuxièmes acides nucléiques sont liés, en un point prédéfini de leur structure, aux éléments plans (10).

13. Procédé selon l'une quelconque des revendications précédentes, dans lequel le modèle partiel se présente sous la forme d'un code à barres.

14. Procédé selon l'une quelconque des revendications précédentes, dans lequel le modèle partiel se présente sous la forme d'une trame.

15. Procédé selon l'une quelconque des revendications précédentes, dans lequel les éléments plans (10) se présentent sous une forme ronde, de préférence avec un diamètre inférieur à 100 µm.

16. Procédé selon l'une quelconque des revendications 3 à 15, dans lequel les quatrièmes acides nucléiques sont mis en contact avec le marquage (32) par distribution homogène par addition par goutte, aspiration, pulvérisation ou atomisation.

17. Procédé selon l'une quelconque des revendications précédentes, dans lequel le procédé de détection en une étape est réalisé en utilisant l'effet suivant :
formation ou séparation d'une paire de donneur/accepteur.

18. Procédé selon l'une quelconque des revendications précédentes, dans lequel le marquage contient le premier acide nucléique en quantité au maximum de 10 µg.
